# EUROPEAN PATENT SPECIFICATION

(11) **EP 1 372 461 B1**
(45) Date of publication and mention of the grant of the patent: **17.11.2010**
(21) Application number: 02719474.5
(22) Date of filing: 28.03.2002
(51) Int. Cl.: A61B 1/247, A61B 1/05

(54) **DENTAL VIDEO IMAGING SYSTEM**
DENTALES VIDEO BILDDARSTELLUNGS SYSTEM
SYST ME D'IMAGERIE VID O DENTAIRE

(30) Priority: 06.04.2001 US 827997; 07.04.2001 WO PCT/US01/11475
(43) Date of publication of application: 02.01.2004
(73) Proprietor: Gendex Corporation, Washington, DC 20006-1813 (US)
(72) Inventor: COOPER, David, Carlsbad, CA 92009 (US)
(74) Representative: Hartz, Nikolai
(86) International application number: PCT/US2002/011160
(87) International publication number: WO 2003/026498

(56) References cited:
- US-A- 5 527 261
- US-A- 5 527 262
- US-A- 5 702 249
- US-A- 6 043 839

## Description

### BACKGROUND OF THE INVENTION

The present invention is related generally to the field of video imaging systems. Specifically, the present invention is related to a dental video imaging system that has camera control unit (CCU) circuitry for the video camera incorporated within the camera handpiece.

Video cameras for imaging dental anatomy are well known in the market place and described in the prior art. For example, Cooper in US 5,702,249 describes DENTSPLY's Acucam® Dental Imaging Camera. These imaging cameras consist of a handpiece having a distal end that contains optics and a sensor assembly for acquiring an image of dental anatomy either intra- or extra-orally. The acquired image is typically transmitted to a docking module for a docking station or directly to an interface unit using a cable with 16 or more wires for subsequent processing before the image is displayed to the clinician. The length of the cable between the handpiece and the docking module or the interface unit is usually restricted to 2 or 3 meters before the degradation of sensor pulses starts to substantially affect the quality of the image.

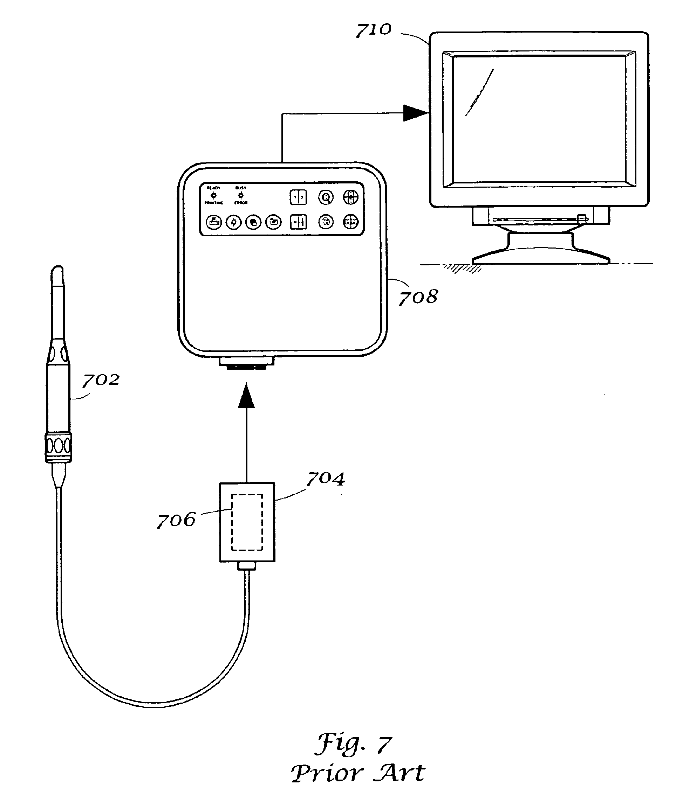

Figure 7 illustrates an arrangement of a docking module and docking station as used with a conventional camera. The conventional camera 702 is connected to a docking module 704 that contains a CCU 706. The docking module 704 with the CCU 706 is then plugged into a docking station 708. The docking station 708 is connected to a video monitor 710 for the clinician to view the images acquired with the conventional camera 702. Alternatively, the conventional camera 702 can be connected to an interface unit (not shown) that has a CCU 706. The interface unit would then be connected to the video monitor 710 for the clinician to view the images acquired with the conventional camera 702. Further, since much of the imaging needed is intra-oral, it is a continuing goal of the developing technology to miniaturize and make the components as small as possible.

Therefore what is needed is a dental imaging system that has a camera handpiece and associated cables that are reduced in size and can be used at larger distances from a docking station. US-A-5 527 261 describes a system according to the preamble of present claim 1.

### SUMMARY OF THE INVENTION

The present invention is directed to a video camera imaging system for intra- and extra-oral imaging of dental anatomy. The dental video imaging system includes a housing having a handle portion and a distal end portion. The distal end portion has a view port for viewing intra- and extra-oral dental anatomy. An optical system is mounted in the distal end of the housing for acquiring, orienting and transmitting an image of the dental anatomy appearing in said view port. A sensor assembly, mounted in the distal end of the housing, converts images received through the optical system into video data signals. A camera control unit (CCU) or control circuit is mounted in the handle portion of the housing. The CCU includes a digital signal processor for receiving the video data image signal from the sensor and may have additional circuitry for providing an S-video, composite video or digital video signal output of the images. The dental video imaging system also includes a utility cable having cable components for conveying utilities (power and/or light) and control signals to the housing and for conveying video output data signals out of the CCU. Flexible cables are used for flexibly interconnecting the CCU with the sensor assembly and the utility cable. A docking station is also included for connecting the utility cable with the corresponding utilities and control signals and for receiving video output data signals for display or further processing.

A key feature and advantage of the present invention is the inclusion of the CCU within the handle portion of the handpiece to provide an S-video, composite video or digital video output, which output reduces the number of cable conductors and connections necessary for connecting the handpiece to a docking station. This arrangement results in a smaller cable of, for example, two to four connector wires, rather than the sixteen or more wires as has been previously required to output image data to a docking station or interface unit from a handpiece. In addition, this arrangement also permits the use of a longer camera cable between the handpiece and the docking station, which camera cable had been previously generally restricted to 2-3 meters.

Another advantage of the present invention is that the handpiece can be connected to a docking station or interface device by a simple connector without the need for a plug-in module containing the CCU.

Still another key feature and advantage of the present invention is that the CCU in the housing is interconnected with a CCD camera or other video sensor and a utility cable by means of flexible cables, preferably flexible printed circuits, or equivalent rigid connectors in such a manner that the CCD or sensor may be translated axially for focusing the desired dental object on the CCD or sensor.

Other features and advantages of the present invention will be apparent from the following more detailed description of the preferred embodiment, taken in conjunction with the accompanying drawings which illustrate, by way of example, the principles of the invention.

### BRIEF DESCRIPTION OF THE DRAWINGS

The present invention is described in greater detail below with reference to the following drawings.

Figure 1 is a plan view of an embodiment of the dental camera of the present invention for intra- or extra-oral imaging of desired objects of dental anatomy.

Figure 2 is a partial section elevational view of the dental camera of Figure 1 showing an arrangement of the CCD and CCU components of the present invention.

Figure 3 is an enlarged elevational cross sectional view of the focusing arrangement of Figure 2.

Figure 4 is a partial sectional elevational view of a second embodiment of a focusing arrangement of the present invention.

Figure 5 is a schematic partial sectional view of an alternate embodiment of the focusing arrangement of Figure 3.

Figure 6 is a schematic view of a video imaging system of the present invention.

Figure 7 is a schematic view of a prior art video imaging system.

Figures 8 and 9 are alternate embodiments of the dental camera of the present invention.

Whenever possible, the same reference numbers will be used throughout the figures to refer to the same parts.

### BRIEF DESCRIPTION OF THE PREFERRED EMBODIMENT

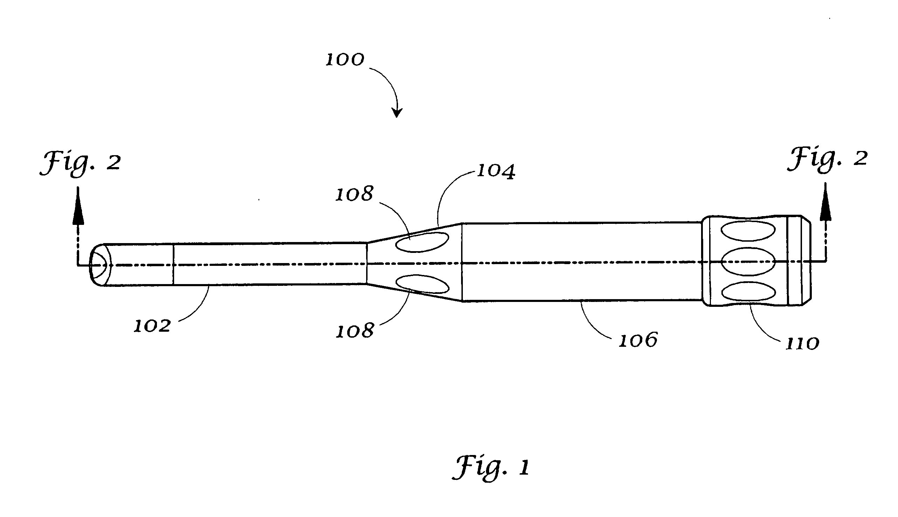

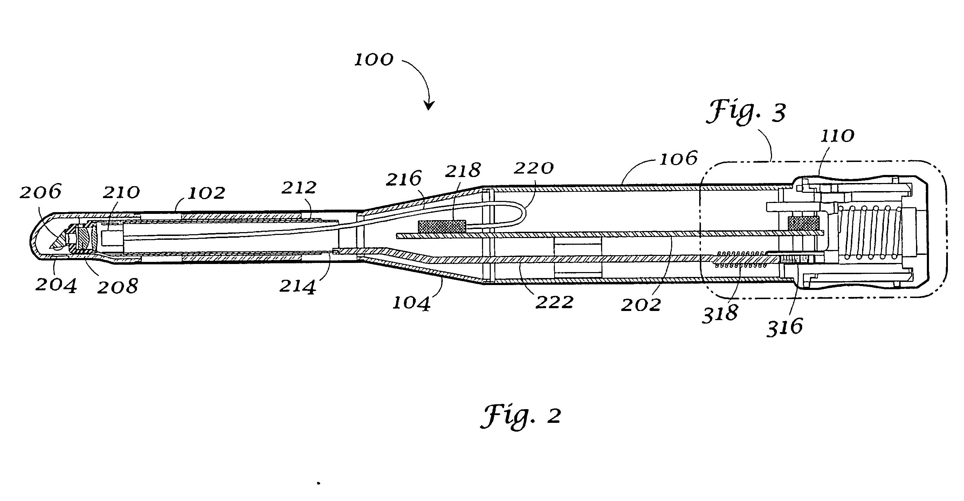

Figures 1 and 2 illustrate an embodiment of a camera head or handpiece 100 of the present invention. The camera head 100 is preferably constructed using stainless steel components, however, other suitable materials can be used. The various body components of the camera head 100 are connected together to provide a watertight casing and are preferably epoxied or glued together.

A distal end of the camera head 100 includes a nose piece 102 that is gently curved at the tip for patient comfort. The nose piece 102 is preferably stainless steel, however, any other suitable material can be used for the nose piece 102. The nose piece 102 is connected via a cone section or transitional portion 104 to a larger diameter handle region 106. The handle region 106 is configured to provide the user with an improved grasping area and a superior torque characteristic and is preferably about 20 mm in diameter.

The cone section 104 is positioned between the nose piece 102 and the handle region 106 and includes four dimples 108, which are spaced about 90° apart, to provide an orientation guide for the clinician. When normally held, the clinician's index finger is located about 45° clockwise of vertical. The dimples 108 are positioned to aid orientation of the camera head 100, as follows:

| **Dimple Position** | **Direction of View** |
|---|---|
| 1:30 o'clock | down |
| 4:30 o'clock | right |
| 7:30 o'clock | up |
| 10:30 o'clock | left |

A focus mode ring 110 is located at the rear of the handle region 106 and is used to select one of the following mode presets:

| **Mode** | **Position** | **Depth of Focus** | **Application** |
|---|---|---|---|
| E | Fully CCW | 40mm to Infinity | Extra-oral use |
| W | 1^{st} detent | 12mm to 40mm | Wide angle exam mode |
| C | 2^{nd} detent | 8mm to 12mm | Close up exam mode |
| Macro | Continuous | 1mm to 8mm | Adjustable focus |

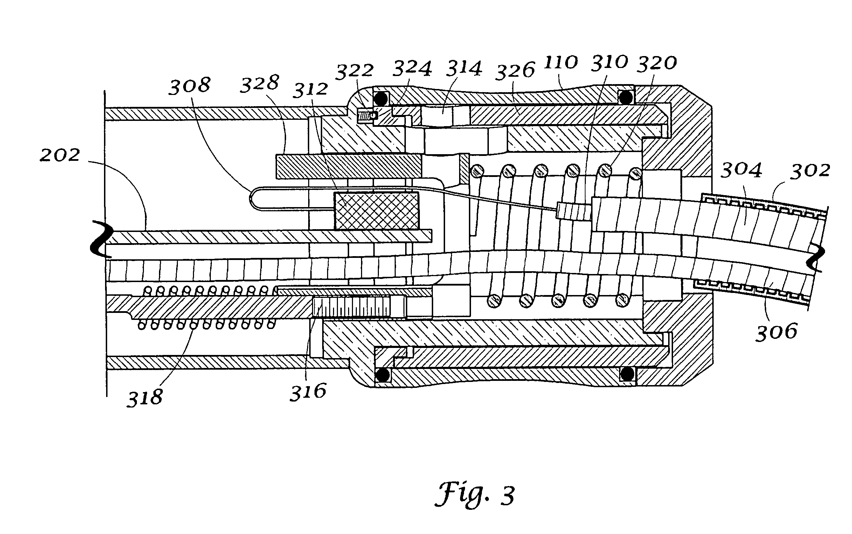

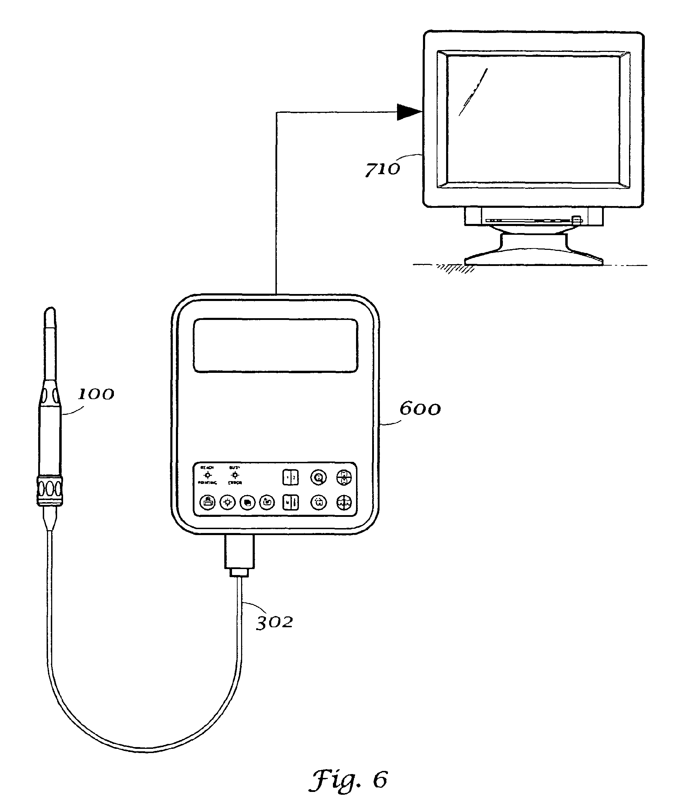

Figure 6 illustrates the camera head 100 being connected to a docking station or interface unit 600 via a small diameter connecting or utility cable 302. The docking station or interface unit 600 can then be connected to the video monitor 710 for display of the images acquired by the camera head or handpiece 100. The utility cable 302 preferably includes a spiral winding to strengthen the utility cable 302 and support any wires, fiber optic light guides, cables or conductors located therein. The spiral winding is preferably a steel spiral winding. In the embodiment shown in Figure 3, the utility cable 302 and the wires, fiber optic light guide, cables and conductors located therein are rigidly connected to the camera handpiece 100. However, in another embodiment of the present invention, the utility cable 302 and the wires, fiber optic light guide, cables and conductors located therein are detachably connected to the camera handpiece 100.

The docking station or interface unit 600 can be any type of device that can provide the camera head 100 with the appropriate power, control and/or light for operation and can then receive the image output signals from the camera head 100 for any additional processing and display. The diameter of the connecting cable 302 is between about 0.125 and about 0.5 inches and is preferably about 0.25 inches. A smaller diameter connecting cable 302 is possible because of a control circuit or camera control unit (CCU) board 202 located in the camera head 100 as shown in Figure 2. The CCU board 202 preferably provides an S-video output signal to a camera cable 304, located in the utility cable 302, having 4 wires for the conveying or transmitting the output signal, in contrast to the normal 16 or more wires which are usually required to convey or transmit an image output signal to a CCU located in or interface unit.

In conventional cameras, where the camera head sensor is connected to a CCU in a docking module that is plugged into a docking station or in an interface unit, the camera cable length is usually restricted to 2 or 3 meters. In contrast, in the case of the present invention, the S-video output signal from the CCU board 202 and camera cable 304 have no such restriction and the length of the connecting cable 302 is only restricted by the practical light guide limitation in fiber optic light guide 306 (see Figure 3) also located in connecting cable 302.

The CCU board 202 is a printed circuit board with a size and shape to enable its location within the camera head 100 and handle region 106. The CCU board 202 is preferably manufactured by Matsushita Communications, Inc. (MCI). The maximum permissible width of the CCU board 202 is 17 mm for location within a handle region 106 having a 20 mm outside diameter (OD). A discussed above, a 20 mm OD for the handle region 106 is preferable for optimum user comfort and control. As shown in Figure 2, the distal end of the CCU board 202 is tapered to fit within the conical section 104 that connects the nose piece 102 to the handle region 106. This provides additional area for the CCU board 202 without extending the length of the camera head 100.

The camera control unit board 202 includes a signal processor, which processes the signal from the image sensor 210. The signal processor is preferably a Digital Signal Processor (DSP), but can be an Analog Signal Processor. The CCU board 202 then generates the preferred S-video output signal from the CCU board 202 either from the signal processor or from additional circuitry on the CCU board 202. Additionally, in other embodiments the CCU board 202 can generate a component video or digital video output signal. It is to be understood that the wires in the camera cable 304 to transmit or convey the output signal correspond to the particular type of output signal from the CCU board 202. The signal processor is configured and adjusted by digital commands from a computer via 3 wires in the camera cable 304. This enables the CCU board 202 to be adjusted after the camera head 100 has been assembled and closed. Non-volatile memory on the CCU board 202 can be used to store the adjustments, thereby making the adjustments permanent.

As described above, the connecting or utility cable 302 includes the camera cable 304, which is preferably shielded, and the fiber optic light guide 306. The camera cable 304 is connected to a flexible printed circuit 308 by an encapsulated connection 310 in the camera head 100. The flexible printed circuit 308 is then connected to the CCU board 202 by a connector 312. The camera cable 304 includes wires or conductors to convey or transmit the following signals and information:
- power to the CCU board 202;
- computer control data to the CCU board 202; and
- video output signals from the CCU board 202.

Referring back to Figure 2, an image enters an optical system through a window 204 into a negative lens and a non-inverting prism 206. The window 204 is preferably sapphire, but can also be glass or acrylic. The non-inverting prism 206 is preferably a roof prism or double prism and reflects the image into a main lens assembly 208. The roof prism 206 is angled to obtain a 97.5° direction of view with respect to the optical axis. The negative lens enables a relatively wide angle of view of 62° horizontally through the window 204 by converting the wide angle to a narrow angle for the roof prism 206. This enables a wider viewing angle than can otherwise pass through the prism. It is to be understood that different directions of view and angles of view can be obtained by selecting the appropriate roof prism 206 and/or negative lens.

In another embodiment of the present invention, a simple inverting prism can be used in place of the non-inverting roof prism 206. In this embodiment, an inverted image is sent to the CCU board 202, which includes the appropriate circuitry to orient the image. In still another embodiment of the present invention, the use of the negative lens is not required, and a wide angle image can be sent by the optical system to the CCU board 202.

The main lens assembly 208 is housed in a barrel or enclosure that is connected to a thin-wall lens tube 212, preferably by gluing with epoxy. The lens tube 212 is held in place within the nose piece 102 by a lens holder spacer. The lens tube 212 is preferably stainless steel, although other suitable materials can be used. The preferred embodiment of the main lens assembly 208 includes three elements: a plano-convex lens; a doublet; and a simple lens. The simple lens focuses the image onto an image sensor 210. The image sensor 210 is preferably a ¼-inch, high resolution CCD. However, other types of images sensors can be used such as CMOS devices and active pixel sensors (APS).

To supply light to the camera head 100, one end of the fiber optic light guide 306 terminates in a metal ferrule at the center of a modified Lemo connector (not shown), which passes through the docking station receptacle and enters a port in a light source.

In the connecting cable 302, the light guide 306 is covered by a thin plastic sheath, which continues into the handle portion or region 106 of the camera head 100. In the handle region 106, the light guide 306 separates or bifurcates into two equal bundles (not shown), which pass through the nose piece 102 on either side of the lens tube 212 that holds the main lens assembly 208 via curved slots in the lens holder spacer and terminate on either side of the window 204. By splitting the light guide 306 into two bundles, a more uniform illumination for the optical system can be provided than with a single light guide.

At the tip of the distal end, the light guide bundles are covered in a thin-wall opaque sheath or paint, to prevent light from entering the optical system, and terminate in a ferrule that is glued into the exit ports of the distal end. The ferrules are shaped in such a way as to ensure that the angle of illumination corresponds to the direction of view of the optical system.

In another embodiment of the present invention, a thin-wall opaque barrier is placed between the optical system and the light guides to block stray light and prevent light from the light guide bundles from entering the optical system. The opaque barrier is preferably a metal material, however, the barrier can be made of any suitable material such as plastic, paper or other material.

The image sensor 210 is supported in a sensor tube 214, which is preferably stainless steel. The sensor tube 214 is permitted to slide within the lens tube 212 that holds the optical system and main lens assembly 208. The image sensor 210 is connected by a flexible printed circuit 216 to a connector 218 on the CCU board 202 located in the camera head 100, as shown in Figure 2.

In order to adjust the focus mode, the position of the sensor tube 214 and the image sensor 210 relative to the main lens assembly 208 is adjusted by movement of a focus rod 222 connected to the sensor tube 214. The adjustment of the focus mode is accomplished by rotating the focus mode ring 110 at the rear of the handle region 106, as detailed in Figure 3. Furthermore, the flexible printed circuit 216 has a service loop 220 over the CCU connector 218, so that the loop length for the flexible circuit 216 can adjust when the image sensor 210 and sensor tube 214 are moved by the focus rod 222.

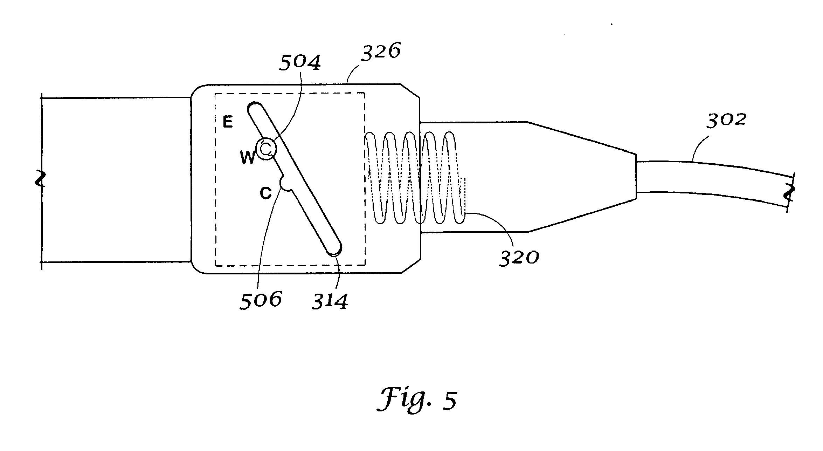

A focus mode assembly includes the focus mode ring 110, which is connected, preferably by gluing, to an inner focus ring 322 with an angled slot 314. When the focus mode ring 110 is adjusted, the inner focus ring 322 with the angled slot 314 causes a pin 504 (see Figure 5) inserted into angled slot 314 to move in the direction of the camera axis. The pin 504 is attached to a linear ring 328 that is connected to one end of the focus rod 222, which focus rod 222 is then connected to the sensor tube 214 at the other end. Therefore, movement of the focus mode ring 110 results in the sensor tube 214 and the sensor 210 to moving axially, thereby changing the sensor's position relative to the main lens assembly 208 and thereby changing the sensor's focal condition.

Due to tolerances in the lenses of the optical system, the positioning of the sensor 210 relative to the optical system has to be adjusted to obtain an optimal focal length for the focus modes. A focus calibration screw 316 and calibration spring 318 are used to adjust the length of the focus rod 222, so that the position of the sensor 210 relative to the optical system can compensate for the lens tolerances. The calibration spring 318 is positioned around the focus rod 222 and is used to prevent any backlash of the focus rod 222 during the adjustment of the focus rod 222 by the focus calibration screw 316. The calibration of the focus rod 222 to compensate for lens tolerance with the focus calibration screw 316 is typically completed during the manufacturing of the camera head 100. In addition, a focus spring 320 is used to apply a tension to the linear ring 328 and pin 504 to maintain the pin 504 in contact with a side of the angled slot 314 and is used to prevent any backlash of the focus mode assembly or the linear ring.

A spring-loaded ball 322 drops into a detent 324 in the focus mode assembly, providing tactile preset focus positions for each of the E, W and C modes discussed above. This enables the clinician to select the desired mode by feel, without the need to take his eyes off the patient. Each mode has sufficient depth of focus so that further fine adjustment within the mode is not necessary.

In another embodiment of the present invention, as shown schematically in Figure 5, a spiral slot 314 in the side of the inner focus ring 326 interacts with the focus pin 504 and linear ring 328, which linear ring 328 is then connected to the focus rod 222. The focus rod 222, as described above, is connected to the sensor tube 214 to move the sensor tube 214 longitudinally or axially as the focus mode ring 110 is rotated. Instead of the spring-loaded ball 322 and detent 324 technique shown in Figure 3 to select the focus modes, notches 506 in the spiral slot 314 corresponding to the W and C focus mode positions are pushed against by a spring loaded linear ring 328 and pin 504. The spring loading is provided by the focus spring 320 and serves two functions: it prevents backlash in the focus adjustment; and it provides a detent action caused by the focus pin 504 being forced to engage the notches 506 as the inner focus ring 326 is rotated. In addition, the E focus mode position is established or located at one end of the spiral slot 502.

In another embodiment of the present invention, the focus rod 222 can move one or more elements of the optical system relative to the image sensor 210 to obtain the different focus modes. The focus rod 222 can be connected to one or more elements of the optical system such as the negative lens or an element in the main lens assembly 208. The adjustment of the focus rod 222 for the different focus modes is accomplished in a manner similar to that described above. The adjustment of the one or more elements in the optical system is performed relative to the sensor 210 to obtain the different focus modes, in contrast to the technique for obtaining the different focus modes described above wherein the sensor 210 is moved relative to the optical system.

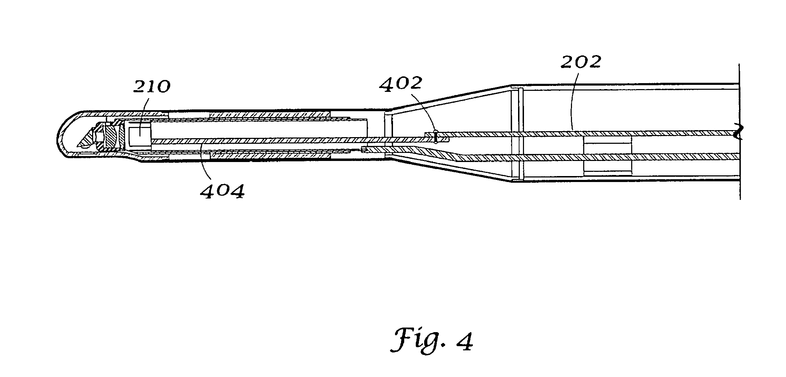

Figure 4 shows a second embodiment of the present invention where the image sensor 210 is connected to an extension board 404, which is rigidly connected to the CCU board 202 by a connecting arrangement 402. The connecting arrangement 402 can include any type of connection that permits axial translation of the image sensor 210 and extension board 404 without detaching from the CCU board 202. The rigidly connected image sensor 210, extension board 404 and CCU board 202 move in tandem with the CCU board 202 being connected to the camera cable 304 as previously described. The adjustment of the focus modes occurs based on the axial movement of the CCU board 202 and image sensor 210 in response to the adjustment of the focus mode ring 110 as described above except that no focus rod 222 is used and the linear ring 328 is connected to the CCU board 202. Movement of the CCU board 202 relative to the utility cable 302 is provided by the service loop in the flexible cable 308 as shown in Figure 3. In another embodiment of the present invention, the extension board 404 can be an included and integral part of the CCU board 202.

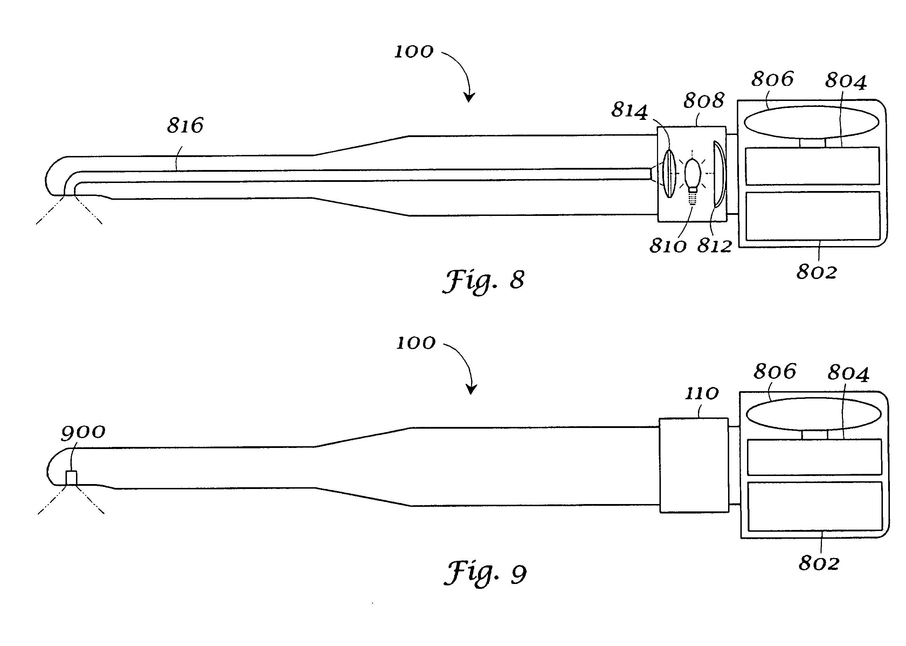

Figure 8 illustrates an alternate embodiment of the present invention, where the utility cable 302 has been replaced by a utility and communication unit. The utility and communication unit can include several different systems to satisfy all of the utility and communication requirements of the camera handpiece 100. The utility and communication unit can be detachably connected to the camera handpiece 100. The utility and communication unit can include a battery 802 to supply the power requirements of the CCU board 202 and other systems and devices in the camera handpiece 100 and the utility and communication unit. The utility and communication unit also includes a transmitter unit 804 and antenna 806 to transmit the video output signal from the CCU board 202. The transmitter unit 804, in one embodiment, may include a receiver to receive transmissions with control instructions and other information for the CCU board 202. The transmitter unit 804 and the antenna 806 can receive their power from the battery 802. The transmitter unit 804 can transmit the video output signals using any standard wireless transmission technique, including infrared and RF transmissions, to a receiver that is preferably connected to a computer, monitor, docking station, interface unit, etc. The utility and communication unit has a light or illumination source 808 to satisfy the lighting requirements of the camera head 100. The light source 808 includes a lamp 810, a reflector 812, and a lens 814. The lens 814 transmits the light to a light guide 816, which is used to transmit the light to the tip of the nose piece 102. The battery 802 can preferably be used to supply the required power to the light source 808.

Figure 9 illustrates another embodiment of the camera handpiece 100 wherein the light source 808 is replaced with one or more light emitting diodes (LEDs) 900 located in the tip of the nose piece 102. The LEDs 900 can be white LEDs or a combination of colored LEDs and can be placed in the exit ports at the distal end and have the same opaque barrier or sheaths to prevent light from entering the optical system as described above. Alternatively, the LEDs 900 can be located elsewhere in the camera handpiece 100, e.g. in the handle region 106, and the light transmitted to the distal end of the camera handpiece 100 by a light guide.

While the invention has been described with reference to a preferred embodiment, it will be understood by those skilled in the art that various changes may be made and equivalents may be substituted for elements thereof without departing from the scope of the invention. In addition, many modifications may be made to adapt a particular situation to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A dental imaging system, comprising:
(a) a handpiece (100) including a handle portion (106) and a distal end portion (102), said distal end portion (102) having a view port (204) for viewing intra-and extra-oral dental anatomy;
(b) an optical system mounted in said distal end portion (102) of said handpiece (100), said optical system being configured to acquire, orient and transmit an image of said dental anatomy appearing in said view port (204);
(c) a sensor assembly (210) mounted in said distal end (102) of said handpiece (100), said sensor assembly (210) being configured to convert images received through said optical system into video data signals;
(d) a camera control unit (202) mounted in said handle portion (106) of said handpiece (100), said camera control unit (202) being configured to receive said video data signals from said sensor assembly (210) and to generate video output signals of said image from said video data signals; and
(e)
an interface means detachably connected to said camera control unit (202) for providing power to said camera control unit (202) and for receiving video output signals from said camera control unit (202) for transmission to a device for display or further processing; **characterised in that**
(f) a connector (218) is disposed in said handpiece to flexibly interconnect said camera control unit (202) with said sensor assembly (210) to permit axial translation of said sensor assembly for focusing a desired dental object on the sensor assembly.

2. The dental imaging system of claim 1 where said interface means comprises a utility cable (302), said utility cable (302) connecting said handpiece (100) to one of an interface unit and a docking station (600), said utility cable (302) comprises a camera cable (304) having first cable components to convey said power to said camera control unit (202) from said one of an interface unit and a docking station (600) and second cable components to convey said video output signals from said camera control unit (202) to said one of an interface unit and a docking station (600).

3. The dental imaging system of claim 2 wherein said second cable components comprise conductors to convey one of S-video signals, composite video signals and digital video signals.

4. The dental imaging system of claim 2 wherein said utility cable further comprises a fiber optic light guide (306) to transmit light from said one of an interface unit and a docking station (600) to said handpiece (100).

5. The dental imaging system of claim 2 wherein said camera cable (304) comprises third cable components to convey control signals to said camera control unit (202) from said one of an interface unit and a docking station (600).

6. The dental imaging system of claim 1 wherein said interface means comprises a battery (802) to supply power to said camera control unit (202).

7. The dental imaging system of claim 1 wherein said interface means comprises a wireless transmission system (804) to transmit said video output signals from said camera control unit (202) to a remote receiver.

8. The dental imaging system of claim 1 further comprises an illumination source (808).

9. The dental imaging system of claim 8 wherein said illumination source (808) comprises a lamp (810), a reflector (812) and a lens (814).

10. The dental imaging system of claim 9 wherein said handpiece (100) further comprises a light guide (816) to receive light from said lens (814) and transmit said light to said distal end portion (102) of said handpiece (100).

11. The dental imaging system of claim 8 wherein said illumination source (808) comprises at least one light emitting diode (900).

12. The dental imaging system of claim 1 further comprising one of an interface unit and a docking station (600) to receive said video output signals from said camera control unit (202).

13. The dental imaging system of claim 1 further comprising:
a connector (312) to flexibly interconnect said interface means and said camera control unit circuit (202).

14. The dental imaging system of claim 13 wherein:
said connector (218) to flexibly interconnect said camera control unit (202) with said sensor assembly (210) comprises a flexible printed circuit (216); and
said connector (312) to flexibly interconnect said interface means and said camera control unit (202) comprises a flexible printed circuit (308).

15. The dental imaging system of claim 14 wherein said flexible printed circuit (216) to interconnect said camera control unit (202) with said sensor assembly (210) is positioned in an elongated loop (220) to permit axial adjustment of said sensor assembly (210) for focusing on said dental anatomy.

16. The dental imaging system of claim 1 wherein said connector (218) to flexibly interconnect said camera control unit (202) with said sensor assembly (210) comprises an extension board (404) rigidly connected to said camera control unit (202) and said sensor assembly (210) such that said camera control unit (202) and said sensor assembly (210) are axially adjustable in tandem for focusing said sensor (210) assembly upon said dental anatomy.

17. The dental imaging system of claim 1 wherein said video output signals from said camera control unit (202) comprise one of S-video signals, composite video signals and digital video signals.

18. The dental imaging system of claim 1 wherein said camera control unit (202) comprises one of a digital signal processor and an analog signal processor.

19. The dental imaging system of claim 1 wherein said sensor assembly (210) comprises one of a charge coupled device, a CMOS device and an active pixel sensor.

20. The dental imaging system of claim 1 wherein said sensor assembly (210) is axially adjustable into a plurality of predetermined focus modes to focus on said dental anatomy.

21. The dental imaging system of claim 20 wherein each of said plurality of predetermined focus modes is selected by adjusting a ring (110) in said handle region (106) of said handpiece (100), said ring (110) being operatively connected to an assembly for axial adjusting said sensor assembly (210) such that an adjustment in said ring (110) results in said assembly axially adjusting said sensor assembly (210) to correspond to a predetermined focus mode.

22. The dental imaging system of claim 1 wherein a plurality of predetermined focus modes are selected by adjusting a ring (110) in said handle region of said handpiece (100), said ring (110) being operatively connected to an assembly for axial adjusting at least one element of said optical system relative to said sensor assembly (210) such that an adjustment in said ring (110) results in said assembly (210) axially adjusting said at least one element of said optical system to correspond to a predetermined focus mode.

23. The dental imaging system of claim 1 wherein said handpiece (100) comprises a conical section (104) between said handle portion (106) and said distal end portion (102), said conical section (104) having plurality of orientation dimples (108) spaced circumferentially about said conical section (104), wherein holding said conical section (104) of said housing (100) with an index finger positioned in an orientation dimple (108) orients said view port in a pre-selected viewing direction.

24. The dental imaging system of claim 2 wherein said optical system comprises:
a window (204) to acquire said image of said dental anatomy;
a prism (206) to reflect said image of said dental anatomy acquired from said window (204); and
a lens assembly (208) to transmit said image of said dental anatomy received from said prism (206) to said sensor assembly (210).

25. The dental imaging system of claim 24 wherein said optical system further comprises a negative lens.

26. The dental imaging system of claim 24 wherein said prism (206) is a non- inverting prism.

27. The dental imaging system of claim 1 wherein said interface means further comprises means for conveying control signals to said camera control unit (202).

28. The dental imaging system of claim 27 wherein the interface means comprises a cable interface to connect a utility cable (302) to said handpiece (100).

29. The dental imaging system of claim 7 wherein said wireless transmission system comprises a transmitter (804) and an antenna (806).

30. The dental imaging system of claim 13 wherein said flexible printed circuit (216) to interconnect said camera control unit (202) with said sensor assembly (210) is positioned in an elongated loop (220) to permit axial adjustment of said sensor assembly (210) for focusing on said dental anatomy.

31. The dental imaging system of claim 24 wherein:
said prism (206) is an inverting prism; and
said camera control unit (202) comprises orienting circuitry to orient said image received from said sensor assembly (210).

32. The dental imaging system of claim 1 wherein said camera control unit (202) comprises a non-volatile memory to store control instructions for said camera control unit (202).

33. A dental imaging system of claim 1, further comprising:
a docking station (600) in wireless communication with said camera control unit (202), said docking station (600) being configured to provide control signals to said camera control unit (202) and to receive video output signals from said camera control unit (202) for display or further processing.

34. The dental imaging system of claim 33 further comprising a utility unit connected to said handpiece (100), said utility unit comprises a power source (802) to provide power to said camera control unit (202), a transmitter unit (804) and antenna (806) to transmit said video output signals from said camera control unit (202) to said docking station (600) and to receive control signals from said docking station (600) for said camera control unit (202), and a light source (808, 900) to provide light to said handpiece (100).

35. The dental imaging system of claim 34 further comprising:
a flexible printed circuit (216) to flexibly interconnect said camera control unit circuit (202) with said sensor assembly (210); and
a flexible printed circuit (308) to flexibly interconnect said utility unit and said camera control unit circuit (202).

36. The dental imaging system of claim 19 or 33 wherein said sensor assembly (210) is a charge coupled device.

37. The dental imaging system of claim 1 or 33 wherein said camera control unit (202) comprises a digital signal processor and means for generating a video output signal in an S-video format.

38. The dental imaging system of claim 1 or 33 wherein said optical system comprises:
a window (204) to acquire said image of said dental anatomy;
a negative lens to pass a narrow angle image of said acquired image;
a non-inverting prism (206) to reflect said narrow angle image of said dental anatomy passed by said negative lens; and
a lens assembly (208) to transmit said narrow angle image of said dental anatomy received from said prism (206) to said sensor assembly (210).

39. A dental imaging system of claim 1, wherein said interface means comprises a cable interface to detachably connect a utility cable (302) to said camera handpiece (100).

## Patentansprüche

1. Dentales Bilddarstellungssystem, umfassend:
(a) ein Handstück (100), welches ein Griffteil (106) und ein distales Endteil (102) einschließt, wobei das Endteil (102) ein Sichtfenster (204) zur Betrachtung der intra- und extraoralen dentalen Anatomie aufweist;
(b) ein optisches System, welches in dem distalen Endteil (102) des Handstücks (100) angebracht ist, wobei dieses optische System konfiguriert ist, ein Bild der dentalen Anatomie, welches in dem Sichtfenster (204) erscheint, aufzunehmen, auszurichten und zu übermitteln;
(c) eine Sensoreinheit (210), welche am distalen Ende (102) des Handstücks (100) angebracht ist, wobei diese Sensoreinheit (210) konfiguriert ist, Bilder, die durch das optische System empfangen wurden, in Videodatensignale umzuwandeln;
(d) eine Kamerakontrolleinheit (202), die in dem Griffteil (106) des Handstücks (100) angebracht ist, wobei diese Kamerakontrolleinheit (202) konfiguriert ist, die Videodatensignale von der Sensoreinheit (210) zu empfangen und von dem Bild Videoausgangssignale aus den Videodatensignalen zu erzeugen; und
(e) eine Schnittstellenvorrichtung, welche abtrennbar mit der Kamerakontrolleinheit (202) verbunden ist, um die Kamerakontrolleinheit (202) mit Strom zu versorgen und Videoausgangssignale von der Kamerakontrolleinheit (202) zu empfangen für die Übertragung an ein Gerät zur Anzeige oder weiteren Verarbeitung;
**dadurch gekennzeichnet, dass**
(f) ein Anschluss (218) in dem Handstück angeordnet ist, um die Kamerakontrolleinheit (202) flexibel mit der Sensoreinheit (210) zu verbinden, um eine axiale Verschiebung der Sensoreinheit und so die Fokussierung eines gewünschten dentalen Objekts in der Sensoreinheit zu ermöglichen.

2. Das dentale Bilddarstellungssystem gemäß Anspruch 1, worin die Schnittstellenvorrichtung ein Versorgungskabel (302) umfasst, welches das Handstück (100) mit einer Schnittstelleneinheit oder einer Andockstation (600) verbindet, wobei das Versorgungskabel (302) ein Kamerakabel (304) umfasst, welches erste Kabelkomponenten aufweist, um den Strom von der Schnittstelleneinheit oder der Andockstation (600) zu der Kamerakontrolleinheit (202) zu übertragen und zweite Kabelkomponenten, um die Videoausgangssignale von der Kamerakontrolleinheit (202) zu der Schnittstelleneinheit oder der Andockstation (600) übertragen.

3. Das dentale Bilddarstellungssystem gemäß Anspruch 2, worin die zweiten Kabelkomponenten Leitungen umfassen, um S-Videosignale, Komposit-Videosignale oder digitale Videosignale zu übertragen.

4. Das dentale Bilddarstellungssystem gemäß Anspruch 2, worin das Versorgungskabel weiterhin einen faseroptischen Lichtleiter (306) umfasst, um Licht von der Schnittstelleneinheit oder der Andockstation (600) an das Handstück (100) zu übertragen.

5. Das dentale Bilddarstellungssystem gemäß Anspruch 2, worin das Kamerakabel (304) dritte Kabelkomponenten enthält, um Steuersignale von der Schnittstelleneinheit oder der Andockstation (600) an die Kamerakontrolleinheit (202) zu übertragen.

6. Das dentale Bilddarstellungssystem gemäß Anspruch 1, worin die Schnittstellenvorrichtung eine Batterie (802) umfasst, um der Kamerakontrolleinheit (202) Strom zu liefern.

7. Das dentale Bilddarstellungssystem gemäß Anspruch 1, worin die Schnittstellenvorrichtung ein drahtloses Übertragungssystem (804) umfasst, um die Videoausgangssignale von der Kamerakontrolleinheit (202) an einen entfernten Empfänger zu übertragen.

8. Das dentale Bilddarstellungssystem gemäß Anspruch 1, das weiterhin eine Beleuchtungsquelle (808) umfasst.

9. Das dentale Bilddarstellungssystem gemäß Anspruch 8, worin die Beleuchtungsquelle (808) eine Lampe (810), einen Reflektor (812) und eine Linse (814) umfasst.

10. Das dentale Bilddarstellungssystem gemäß Anspruch 9, worin das Handstück (100) weiterhin einen Lichtleiter (816) umfasst, um Licht von der Linse (814) zu empfangen und dieses Licht zu dem distalen Endteil (102) des Handstücks (100) zu übertragen.

11. Das dentale Bilddarstellungssystem gemäß Anspruch 8, worin die Beleuchtungsquelle (808) mindestens eine Leuchtdiode (900) umfasst.

12. Das dentale Bilddarstellungssystem gemäß Anspruch 1, weiterhin umfassend eine Schnittstelleneinheit oder eine Andockstation (600), um die Videoausgangssignale von der Kamerakontrolleinheit (202) zu empfangen.

13. Das dentale Bilddarstellungssystem gemäß Anspruch 1, weiterhin umfassend:
einen Anschluss (312), um die Schnittstellenvorrichtung und die Kamerakontrolleinheit (202) flexibel miteinander zu verbinden.

14. Das dentale Bilddarstellungssystem gemäß Anspruch 13, worin:
der Anschluss (218) für die flexible Verbindung der Kamerakontrolleinheit (202) mit der Sensoreinheit (210) eine flexible gedruckte Schaltung (216) umfasst; und der Anschluss (312) für die flexible Verbindung der Schnittstellenvorrichtung mit der Kamerakontrolleinheit (202) eine flexible gedruckte Schaltung (308) umfasst.

15. Das dentale Bilddarstellungssystem gemäß Anspruch 14, worin die flexible gedruckte Schaltung (216) für die Verbindung der Kamerakontrolleinheit (202) mit der Sensoreinheit (210) in einer länglichen Schleife (220) angeordnet ist, um eine axiale Justierung der Sensoreinheit (210) zur Fokussierung auf die dentale Anatomie zu ermöglichen.

16. Das dentale Bilddarstellungssystem gemäß Anspruch 1, worin der Anschluss (218) für die flexible Verbindung der Kamerakontrolleinheit (202) mit der Sensoreinheit (210) eine Erweiterungskarte (404) umfasst, welche fest mit der Kamerakontrolleinheit (202) und der Sensoreinheit (210) verbunden ist, so dass die Kamerakontrolleinheit (202) und die Sensoreinheit (210) gemeinsam axial justierbar sind zur Fokussierung der Sensoreinheit (210) auf die dentale Anatomie.

17. Das dentale Bilddarstellungssystem gemäß Anspruch 1, worin die Videoausgangssignale von der Kamerakontrolleinheit (202) S-Videosignale, Komposit-Videosignale oder digitale Videosignale umfassen.

18. Das dentale Bilddarstellungssystem gemäß Anspruch 1, worin die Kamerakontrolleinheit (202) einen digitalen Signalprozessor oder einen analogen Signalprozessor umfasst.

19. Das dentale Bilddarstellungssystem gemäß Anspruch 1, worin die Sensoreinheit (210) ein ladungsgekoppeltes Bauteil (charge-coupled device; CCD), ein CMOS-Bauteil oder einen aktiven Pixelsensor umfasst.

20. Das dentale Bilddarstellungssystem gemäß Anspruch 1, worin die Sensoreinheit (210) in einer Vielzahl vorbestimmter Fokussierungsmodi axial justierbar ist, um auf die dentale Anatomie zu fokussieren.

21. Das dentale Bilddarstellungssystem gemäß Anspruch 20, worin jede der Vielzahl der vorbestimmten Fokussierungsmodi **dadurch** ausgewählt wird, dass ein Ring (110) in dem Griffteil (106) des Handstücks (100) eingestellt wird, wobei der Ring (110) mit einer Vorrichtung für die axiale Justierung der Sensoreinheit (210) wirkverbunden ist, so dass eine Einstellung des Rings (110) bewirkt, dass die Vorrichtung die Sensoreinheit (210) entsprechend eines vorbestimmten Fokussierungsmodus axial justiert.

22. Das dentale Bilddarstellungssystem gemäß Anspruch 1, worin eine Vielzahl vorbestimmter Fokussierungsmodi **dadurch** ausgewählt werden, dass ein Ring (110) in dem Griffteil des Handstücks (100) eingestellt wird, wobei der Ring (110) mit einer Vorrichtung für die axiale Justierung von zumindest einem Element des optischen Systems relativ zu der Sensoreinheit (210) wirkverbunden ist, so dass eine Einstellung des Rings (110) bewirkt, dass die Vorrichtung zumindest ein Element des optischen Systems entsprechend eines vorbestimmten Fokussierungsmodus axial justiert.

23. Das dentale Bilddarstellungssystem gemäß Anspruch 1, worin das Handstück (100) einen konischen Abschnitt (104) zwischen dem Griffteil (106) und dem distalen Endteil (102) umfasst, wobei der konische Abschnitt (104) eine Vielzahl von Orientierungsvertiefungen (108) aufweist, die ringsum um den konischen Abschnitt (104) angeordnet sind, worin das Halten dieses konischen Abschnitts (104) des Gehäuses (100) mit einem Zeigefinger in einer Orientierungsvertiefung (108) das Sichtfenster in eine vorausgewählte Blickrichtung ausrichtet.

24. Das dentale Bilddarstellungssystem gemäß Anspruch 2, worin das optische System umfasst:
ein Fenster (204), um das Bild der dentalen Anatomie aufzunehmen;
ein Prisma (206), um das Bild der dentalen Anatomie, welches durch das Fenster (204) aufgenommen wurde, zu reflektieren; und
eine Linseneinheit (208), um das Bild der dentalen Anatomie, welches von dem Prisma (206) empfangen wurde, an die Sensoreinheit (210) zu übertragen.

25. Das dentale Bilddarstellungssystem gemäß Anspruch 24, worin das optische System weiterhin eine negative Linse umfasst.

26. Das dentale Bilddarstellungssystem gemäß Anspruch 24, worin das Prisma (206) ein nicht-invertierendes Prisma ist.

27. Das dentale Bilddarstellungssystem gemäß Anspruch 1, worin die Schnittstellenvorrichtung weiterhin Mittel enthält, um Steuersignale an die Kamerakontrolleinheit (202) zu übertragen.

28. Das dentale Bilddarstellungssystem gemäß Anspruch 27, worin die Schnittstellenvorrichtung eine Kabelschnittstelle zur Verbindung eines Versorgungskabels (302) mit dem Handstück (100) umfasst.

29. Das dentale Bilddarstellungssystem gemäß Anspruch 7, worin das drahtlose Übertragungssystem einen Sender (804) und eine Antenne (806) umfasst.

30. Das dentale Bilddarstellungssystem gemäß Anspruch 13, worin die flexible gedruckte Schaltung (216) für die Verbindung der Kamerakontrolleinheit (202) mit der Sensoreinheit (210) in einer länglichen Schleife (220) angeordnet ist, um eine axiale
Justierung der Sensoreinheit (210) zur Fokussierung auf die dentale Anatomie zu ermöglichen.

31. Das dentale Bilddarstellungssystem gemäß Anspruch 24, worin:
das Prisma (206) ein invertierendes Prisma ist; und
die Kamerakontrolleinheit (202) ausrichtende Schaltungen umfasst, um das Bild, welches von der Sensoreinheit (210) empfangen wurde, auszurichten.

32. Das dentale Bilddarstellungssystem gemäß Anspruch 1, worin die Kamerakontrolleinheit (202) einen dauerhaften Speicher umfasst, um Steueranweisungen für die Kamerakontrolleinheit (202) zu speichern.

33. Das dentale Bilddarstellungssystem gemäß Anspruch 1, weiterhin umfassend:
eine Andockstation (600) in drahtloser Kommunikation mit der Kamerakontrolleinheit (202), wobei die Andockstation (600) konfiguriert ist, um die Kamerakontrolleinheit (202) mit Steuersignalen zu versorgen und Videoausgangssignale von der Kamerakontrolleinheit (202) zur Anzeige oder weiteren Verarbeitung zu empfangen.

34. Das dentale Bilddarstellungssystem gemäß Anspruch 33, weiterhin umfassend eine Versorgungseinheit, die mit dem Handstück (100) verbunden ist, wobei diese Versorgungseinheit eine Stromquelle (802) für die Stromversorgung der Kamerakontrolleinheit (202) umfasst, eine Sendereinheit (804) und eine Antenne (806), um die Videoausgangssignale von der Kamerakontrolleinheit (202) zu der Andockstation (600) zu übertragen und um Steuersignale für die Kamerakontrolleinheit (202) von der Andockstation (600) zu empfangen, und eine Beleuchtungsquelle (808, 900), um das Handstück (100) mit Licht zu versorgen.

35. Das dentale Bilddarstellungssystem gemäß Anspruch 34, weiterhin umfassend:
eine flexible gedruckte Schaltung (216), um die Kamerakontrolleinheitsschaltung (202) und die Sensoreinheit (210) flexibel miteinander zu verbinden; und
eine flexible gedruckte Schaltung (308), um die Versorgungseinheit und die Kamerakontrolleinheitsschaltung (202) flexibel miteinander zu verbinden.

36. Das dentale Bilddarstellungssystem gemäß Anspruch 19 oder 33, worin die Sensoreinheit (210) ein ladungsgekoppeltes Bauteil (charge-coupled device; CCD) ist.

37. Das dentale Bilddarstellungssystem gemäß Anspruch 1 oder 33, worin die Kamerakontrolleinheit (202) einen digitalen Signalprozessor und Mittel zur Erzeugung eines Videoausgangssignals im S-Video-Format umfasst.

38. Das dentale Bilddarstellungssystem gemäß Anspruch 1 oder 33, worin das optische System umfasst:
ein Fenster (204), um das Bild der dentalen Anatomie aufzunehmen;
eine negative Linse, die ein Engwinkelbild des aufgenommenen Bildes passieren lässt;
ein nicht-invertierendes Prisma (206), um das Engwinkelbild der dentalen Anatomie, welches durch die negative Linse passiert ist, zu reflektieren; und
eine Linseneinheit (208), um das Engwinkelbild der dentalen Anatomie, welches von dem Prisma (206) ausgehend empfangen wurde, zu der Sensoreinheit (210) zu übertragen.

39. Das dentale Bilddarstellungssystem gemäß Anspruch 1, worin die Schnittstellenvorrichtung eine Kabelschnittstelle umfasst, um ein Versorgungskabel (302) abtrennbar mit dem Kamerahandstück (100) zu verbinden.

## Revendications

1. Système d'imagerie dentaire, comprenant :
(a) un instrument (100) incluant une partie formant poignée (106) et une partie d'extrémité distale (102), ladite partie d'extrémité distale (102) ayant un orifice de visualisation (204) pour voir l'anatomie dentaire intra- et extra-orale ;
(b) un système optique monté dans ladite partie d'extrémité distale (102) dudit instrument (100), ledit système optique étant configuré pour acquérir, orienter et transmettre une image de ladite anatomie dentaire apparaissant dans ledit orifice de visualisation (204) ;
(c) un ensemble formant capteur (210) monté dans ladite extrémité distale (102) dudit instrument (100), ledit ensemble formant capteur (210) étant configuré pour transformer des images reçues par ledit système optique en signaux de données vidéo ;
(d) une unité de commande d'appareil de prise de vues (202) montée dans ladite partie formant poignée (106) dudit instrument (100), ladite unité de commande d'appareil de prise de vues (202) étant configurée pour recevoir lesdits signaux de données vidéo en provenance dudit ensemble formant capteur (210) et pour produire des signaux de sortie vidéo de ladite image à partir desdits signaux de données vidéo ; et
(e) un moyen d'interface relié de façon amovible à ladite unité de commande d'appareil de prise de vues (202) pour fournir de l'énergie à ladite unité de commande d'appareil de prise de vues (202) et pour recevoir des signaux de sortie vidéo en provenance de ladite unité de commande d'appareil de prise de vues (202) pour transmission à un dispositif pour affichage ou traitement ultérieur ;
**caractérisé en ce que**
(f) un connecteur (218) est disposé dans ledit instrument pour relier de manière souple ladite unité de commande d'appareil de prise de vues (202) audit ensemble formant capteur (210) pour permettre la translation axiale dudit ensemble formant capteur pour faire la mise au point d'un objet dentaire souhaité sur l'ensemble formant capteur.

2. Système d'imagerie dentaire selon la revendication 1, dans lequel ledit moyen d'interface comprend un câble utilitaire (302), ledit câble utilitaire (302) reliant ledit instrument (100) à l'une d'une unité d'interface et d'une station d'accueil (600), ledit câble utilitaire (302) comprend un câble d'appareil de prise de vues (304) ayant des premières composantes de câble pour transporter ladite énergie vers ladite unité de commande d'appareil de prise de vues (202) en provenance dudit élément parmi une unité d'interface et une station d'accueil (600) et des secondes composantes de câble pour transporter lesdits signaux de sortie vidéo en provenance de ladite unité de commande d'appareil de prise de vues (202) vers ledit élément parmi une unité d'interface et une station d'accueil (600).

3. Système d'imagerie dentaire selon la revendication 2, dans lequel lesdites secondes composantes de câble comprennent des conducteurs pour transporter soit des signaux S-vidéo, soit des signaux vidéo composites, soit des signaux vidéo numériques.

4. Système d'imagerie dentaire selon la revendication 2, dans lequel ledit câble utilitaire comprend en outre un guide optique de lumière à fibre (306) pour transmettre une lumière provenant dudit élément parmi une unité d'interface et une station d'accueil (600) audit instrument (100).

5. Système d'imagerie dentaire selon la revendication 2, dans lequel ledit câble d'appareil de prise de vues (304) comprend des troisièmes composantes de câble pour transporter des signaux de commande vers ladite unité de commande d'appareil de prise de vues (202) en provenance dudit élément parmi une unité d'interface et une station d'accueil (600).

6. Système d'imagerie dentaire selon la revendication 1, dans lequel ledit moyen d'interface comprend une batterie (802) pour fournir de l'énergie à ladite unité de commande d'appareil de prise de vues (202).

7. Système d'imagerie dentaire selon la revendication 1, dans lequel ledit moyen d'interface comprend un système de transmission sans fil (804) pour transmettre lesdits signaux de sortie vidéo en provenance de ladite unité de commande d'appareil de prise de vues (202) vers un récepteur distant.

8. Système d'imagerie dentaire selon la revendication 1 qui comprend en outre une source d'éclairage (808).

9. Système d'imagerie dentaire selon la revendication 8, dans lequel ladite source d'éclairage (808) comprend une lampe (810), un réflecteur (812) et une lentille (814).

10. Système d'imagerie dentaire selon la revendication 9, dans lequel ledit instrument (100) comprend en outre un guide de lumière (816) pour recevoir la lumière en provenance de ladite lentille (814) et pour transmettre ladite lumière à ladite partie d'extrémité distale (102) dudit instrument (100).

11. Système d'imagerie dentaire selon la revendication 8, dans lequel ladite source d'éclairage (808) comprend au moins une diode électroluminescente (900).

12. Système d'imagerie dentaire selon la revendication 1, comprenant en outre l'une d'une unité d'interface et d'une station d'accueil (600) pour recevoir lesdits signaux de sortie vidéo en provenance de ladite unité de commande d'appareil de prise de vues (202).

13. Système d'imagerie dentaire selon la revendication 1, comprenant en outre :
un connecteur (312) pour relier de manière souple ledit moyen d'interface et ledit circuit d'unité de commande d'appareil de prise de vues (202).

14. Système d'imagerie dentaire selon la revendication 13, dans lequel :
ledit connecteur (218) pour relier de manière souple ladite unité de commande d'appareil de prise de vues (202) audit ensemble formant capteur (210) comprend un circuit imprimé souple (216) ; et
ledit connecteur (312) pour relier de manière souple ledit moyen d'interface et ladite unité de commande d'appareil de prise de vues (202) comprend un circuit imprimé souple (308).

15. Système d'imagerie dentaire selon la revendication 14, dans lequel ledit circuit imprimé souple (218) pour relier ladite unité de commande d'appareil de prise de vues (202) audit ensemble formant capteur (210) est positionné en une boucle allongée (220) pour permettre l'ajustement axial dudit ensemble formant capteur (210) pour faire la mise au point sur ladite anatomie dentaire.

16. Système d'imagerie dentaire selon la revendication 1, dans lequel ledit connecteur (218) pour relier de manière souple ladite unité de commande d'appareil de prise de vues (202) audit ensemble formant capteur (210) comprend une carte d'extension (404) reliée de manière rigide à ladite unité de commande d'appareil de prise de vues (202) et audit ensemble formant capteur (210) de sorte que ladite unité de commande d'appareil de prise de vues (202) et ledit ensemble formant capteur (210) sont ajustables en tandem de façon axiale pour faire la mise au point dudit ensemble formant capteur (210) sur ladite anatomie dentaire.

17. Système d'imagerie dentaire selon la revendication 1, dans lequel lesdits signaux de sortie vidéo en provenance de ladite unité de commande d'appareil de prise de vues (202) comprennent soit des signaux S-vidéo, soit des signaux vidéo composites, soit des signaux vidéo numériques.

18. Système d'imagerie dentaire selon la revendication 1, dans lequel ladite unité de commande d'appareil de prise de vues (202) comprend l'un d'un processeur de signal numérique et d'un processeur de signal analogique.

19. Système d'imagerie dentaire selon la revendication 1, dans lequel ledit ensemble formant capteur (210) comprend l'un d'un circuit à couplage de charges, d'un dispositif CMOS et d'un capteur à pixels actifs.

20. Système d'imagerie dentaire selon la revendication 1, dans lequel ledit ensemble formant capteur (210) est ajustable de façon axiale dans plusieurs modes de mise au point prédéterminés pour faire la mise au point sur ladite anatomie dentaire.

21. Système d'imagerie dentaire selon la revendication 20, dans lequel chaque mode desdits plusieurs modes de mise au point prédéterminés est sélectionné en ajustant une bague (110) dans ladite zone formant poignée (106) dudit instrument (100), ladite bague (110) étant reliée de manière opérationnelle à un ensemble pour l'ajustement axial dudit ensemble formant capteur (210) de sorte qu'un ajustement de ladite bague (110) résulte en ce que ledit ensemble ajuste de façon axiale ledit ensemble formant capteur (210) pour correspondre à un mode de mise au point prédéterminé.

22. Système d'imagerie dentaire selon la revendication 1, dans lequel plusieurs modes de mise au point prédéterminés sont sélectionnés en ajustant une bague (110) dans ladite zone formant poignée dudit instrument (100), ladite bague (110) étant reliée de manière opérationnelle à un ensemble pour l'ajustement axial d'au moins un élément dudit système optique par rapport audit ensemble formant capteur (210) de sorte qu'un ajustement de ladite bague (110) résulte en ce que ledit ensemble (210) ajuste de façon axiale ledit au moins un élément dudit système optique pour correspondre à un mode de mise au point prédéterminé.

23. Système d'imagerie dentaire selon la revendication 1, dans lequel ledit instrument (100) comprend une section conique (104) entre ladite partie formant poignée (106) et ladite partie d'extrémité distale (102), ladite section conique (104) ayant plusieurs cavités d'orientation (108) espacées de manière circonférentielle autour de ladite section conique (104), dans lequel le maintien de ladite section conique (104) dudit logement (100) avec l'index positionné dans une cavité d'orientation (108) oriente ledit orifice de visualisation dans une direction d'observation présélectionnée.

24. Système d'imagerie dentaire selon la revendication 2, dans lequel ledit système optique comprend :
une fenêtre (204) pour acquérir ladite image de ladite anatomie dentaire ;
un prisme (206) pour réfléchir ladite image d'anatomie dentaire acquise à partir de ladite fenêtre (204) ; et
un ensemble formant lentille (208) pour transmettre ladite image d'anatomie dentaire reçue en provenance dudit prisme (206) audit ensemble formant capteur (210).

25. Système d'imagerie dentaire selon la revendication 24, dans lequel ledit système optique comprend en outre une lentille négative.

26. Système d'imagerie dentaire selon la revendication 24, dans lequel ledit prisme (206) est un prisme non inverseur.

27. Système d'imagerie dentaire selon la revendication 1, dans lequel ledit moyen d'interface comprend en outre un moyen pour transporter des signaux de commande vers ladite unité de commande d'appareil de prise de vues (202).

28. Système d'imagerie dentaire selon la revendication 27, dans lequel le moyen d'interface comprend une interface de câble pour relier un câble utilitaire (302) audit instrument (100).

29. Système d'imagerie dentaire selon la revendication 7, dans lequel ledit système de transmission sans fil comprend un émetteur (804) et une antenne (806).

30. Système d'imagerie dentaire selon la revendication 13, dans lequel ledit circuit imprimé souple (216) pour interconnecter ladite unité de commande d'appareil de prise de vues (202) avec ledit ensemble formant capteur (210) est positionné en une boucle allongée (220) pour permettre l'ajustement axial dudit ensemble formant capteur (210) pour faire la mise au point sur ladite anatomie dentaire.

31. Système d'imagerie dentaire selon la revendication 24, dans lequel :
ledit prisme (206) est un prisme inverseur ; et
ladite unité de commande d'appareil de prise de vues (202) comprend des circuits d'orientation pour orienter ladite image reçue en provenance dudit ensemble formant capteur (210).

32. Système d'imagerie dentaire selon la revendication 1, dans lequel ladite unité de commande d'appareil de prise de vues (202) comprend une mémoire non volatile pour stocker des instructions de commande pour ladite unité de commande d'appareil de prise de vues (202).

33. Système d'imagerie dentaire selon la revendication 1, comprenant en outre :
une station d'accueil (600) en communication sans fil avec ladite unité de commande d'appareil de prise de vues (202), ladite station d'accueil (600) étant configurée pour fournir des signaux de commande à ladite unité de commande d'appareil de prise de vues (202) et pour recevoir des signaux de sortie vidéo en provenance de ladite unité de commande d'appareil de prise de vues (202) pour affichage ou traitement ultérieur.

34. Système d'imagerie dentaire selon la revendication 33, comprenant en outre une unité utilitaire reliée audit instrument (100), ladite unité utilitaire comprend une source d'énergie (802) pour fournir de l'énergie à ladite unité de commande d'appareil de prise de vues (202), une unité émettrice (804) et une antenne (806) pour transmettre lesdits signaux de sortie vidéo depuis ladite unité de commande d'appareil de prise de vues (202) à ladite station d'accueil (600) et pour recevoir des signaux de commande en provenance de ladite station d'accueil (600) pour ladite unité de commande d'appareil de prise de vues (202), et une source de lumière (808, 900) pour fournir de la lumière audit instrument (100).

35. Système d'imagerie dentaire selon la revendication 34, comprenant en outre :
un circuit imprimé souple (216) pour interconnecter de manière souple ledit circuit d'unité de commande d'appareil de prise de vues (202) avec ledit ensemble formant capteur (210) ; et
un circuit imprimé souple (308) pour interconnecter de manière souple ladite unité utilitaire et ledit circuit d'unité de commande d'appareil de prise de vues (202).

36. Système d'imagerie dentaire selon la revendication 19 ou 33, dans lequel ledit ensemble formant capteur (210) est un circuit à couplage de charges.

37. Système d'imagerie dentaire selon la revendication 1 ou 33, dans lequel ladite unité de commande d'appareil de prise de vues (202) comprend un processeur de signal numérique et un moyen pour produire un signal de sortie vidéo dans un format S-vidéo.

38. Système d'imagerie dentaire selon la revendication 1 ou 33, dans lequel ledit système optique comprend :
une fenêtre (204) pour acquérir ladite image d'anatomie dentaire ;
une lentille négative pour passer une image angulaire étroite de ladite image acquise ;
un prisme non inverseur (206) pour refléter ladite image angulaire étroite de ladite anatomie dentaire passée par ladite lentille négative ; et
un ensemble formant lentille (208) pour transmettre ladite image angulaire étroite de ladite anatomie dentaire reçue en provenance dudit prisme (206) audit ensemble formant capteur (210).

39. Système d'imagerie dentaire selon la revendication 1, dans lequel ledit moyen d'interface comprend une interface de câble pour relier de façon amovible un câble utilitaire (302) audit instrument d'appareil de prise de vues (100).
